(19) Europäisches Patentamt
European Patent Office
Office européen des brevets

(11) Veröffentlichungsnummer: **0 061 019**
**B1**

(12) **EUROPÄISCHE PATENTSCHRIFT**

(45) Veröffentlichungstag der Patentschrift: **27.12.84**

(51) Int. Cl.³: **C 07 D 239/47**

(21) Anmeldenummer: **82101488.3**

(22) Anmeldetag: **26.02.82**

(54) **Verfahren zur Herstellung von 2-Alkoxy-4-aminopyrimidinen.**

(30) Priorität: **25.03.81 DE 3111613**

(43) Veröffentlichungstag der Anmeldung: **29.09.82 Patentblatt 82/39**

(45) Bekanntmachung des Hinweises auf die Patenterteilung: **27.12.84 Patentblatt 84/52**

(84) Benannte Vertragsstaaten: **AT BE CH DE FR GB IT LI NL SE**

(56) Entgegenhaltungen:
**DE - A - 2 412 854**

**CHEMICAL ABSTRACTS, Vol. 66, No. 13, 27. März 1967, Columbus, Ohio, USA, NITTA YOSHIHIRO, OKUI KIYOSHI, ITO KIYOHIKO "4-Alkoxy-6-aminopyrimidine derivatives" Seite 5247, Spalte 2, Zusammenfassung No. 55 550 f**
**CHEMICAL ABSTRACTS, Vol. 80, No. 19, 13. März 1974, Columbus, Ohio, USA, R.A. KHMEL'NITSKII, N.A. KLYUEV, E.A. KUNIA, A.A. KROPACHEVA "Mass spectra of methoxy derivatives of 4-aminopyrimidines" Seite 330, Spalte 2, Zusammenfassung No. 107 470 g**
**CHEMICAL ABSTRACTS, Vol. 69, No. 21, 18. November 1968, Columbus, Ohio, USA, P.M. PITHA, G.C. Butler,**

(73) Patentinhaber: **DYNAMIT NOBEL AKTIENGESELLSCHAFT, Postfach 1209, D-5210 Troisdorf, Bez. Köln (DE)**

(72) Erfinder: **Peeters, Hermann, Dr., Porzer Strasse 1, D-5216 Niederkassel (DE)**

(56) Entgegenhaltungen: (Fortsetzung)
**"The photolysis of O-methylcytosine in phosphate suiutions" Seite 8025, Spalte 1, Zusammenfassung No. 85 991 w**
**CHEMICAL ABSTRACTS, Vol. 68, No. 25, 17. Juni 1968, Columbus, Ohio, USA, OKANO TEISUKE, GOYA SHUJIRO, MATSUMOTO HITOSHI "Electronic properties of N-heteroaromatics. XV. Synthesis of fluoropyrimidine derivatives" Seite 11041, Spalte 2, Zusammenfassung No. 114 540 k**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

EP 0 061 019 B1

**Beschreibung**

Die vorliegende Erfindung betrifft ein Verfahren zur Herstellung von 2-Alkoxy-4-aminopyrimidinen.

Die Synthese von 4-Amino-2-alkoxypyrimidinen ist bisher aufwendig. Das unsubstituierte 4-Amino--2-alkoxypyrimidin bildet sich aus 2,4-Dichlorpyrimidin durch Umsetzung mit Ammoniak und weiterer Reaktion des 4-Amino-2-chlorpyrimidins mit Alkalimethylat, wobei ein Isomerengemisch entsteht, das nur mit grossem Aufwand trennbar ist [J. Amer. Chem. Soc. 52 (1930) 1152; C.A. 52, 6360 b], sowie durch Hydrierung des nur schwer zugänglichen 4-Amino-6-chlor-2-alkoxypyrimidins [Monatsh. Chem. 94 (1963) 1178] oder durch Reaktion von 2,4-Dimethoxypyrimidin mit Natriumamid in flüssigem Ammoniak [C.A. 58, 5460]. Das in 5-Stellung durch die Hydroxymethylgruppe substituierte 4-Amino-2-methoxypyrimidin ist nur durch Reduktion des schlecht zugänglichen 4-Amino-5-ethoxycarbonylpyrimidins mit Lithiumaluminiumhydrid [J. Org. Chem. 27 (1962) 3614] dargestellt worden.

Der vorliegenden Erfindung lag die Aufgabe zugrunde, aus gut zugänglichen und preiswerten Ausgangsprodukten durch eine einfache Reaktion 2-Alkoxypyrimidine mit hoher Ausbeute zu synthetisieren. Verfahrenswege mit hohem Aufwand, wie Isomerentrennungen, waren zu vermeiden.

Gegenstand der Erfindung ist ein Verfahren zur Herstellung von 2-Alkoxy-4-aminopyrimidinen der Formel

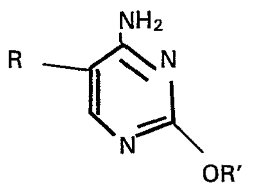

1,

worin R ein Wasserstoffatom, geradkettige, verzweigte oder cyclische Alkylreste mit 1 bis 20 Kohlenstoffatomen, geradkettige oder verzweigte Reste $(CH_2)_nCN$, $(CH_2)_n$-COOR'', $(CH_2)_n$-$NH_2$, $(CH_2)_n$OR'', $(CH_2)_n$-Cyc, wobei Cyc ein isocyclischer oder heterocyclischer Ring mit ein- oder mehrcyclischer Struktur, R'' Alkylreste mit 1 bis 15 Kohlenstoffatomen oder Reste einwertiger Phenole und n = 1 bis 5 ist, und R' geradkettige, verzweigte oder cyclische Alkylreste mit 1 bis 10 Kohlenstoffatomen oder $(CH_2)_n$Cyc, wobei n und Cyc die oben angegebene Bedeutung haben, bedeutet, welches dadurch gekennzeichnet ist, dass ein 4-Amino-2-carboxymethylthiopyrimidin der Formel

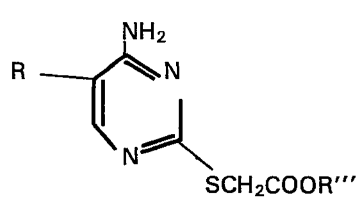

2,

worin R dieselbe Bedeutung wie in der Formel (1) hat und R''' Wasserstoff oder einen geradkettigen, verzweigten oder cyclischen Alkylrest mit 1 bis 10 Kohlenstoffatomen bedeutet mit einem Alkohol der Formel

HOR' 3,

worin R' dieselbe Bedeutung wie in der Formel (1) besitzt, in Gegenwart einer Base umgesetzt wird.

Das Verfahren liefert die Produkte in hoher Reinheit und Ausbeute.

Die 4-Amino-2-carboxymethylthiopyrimidine der Formel (2) sind durch Umsetzung von 4-Amino-2--mercaptopyrimidinen mit Chloressigsäure oder Chloressigsäureestern gut zugänglich [J. Biol. Chem. 177 (1949) 357].

Zur Durchführung der vorliegenden Erfindung wird ein 4-Amino-2-carboxymethylthiopyrimidin (2), wobei als Carboxygruppe sehr bevorzugt die Gruppe -COOH und weiter bevorzugt -$COOCH_3$ und $COOC_2H_5$ eingesetzt wird, mit einem Alkohol in Gegenwart einer Base umgesetzt.

Der Alkohol wird im allgemeinen im Überschuss, bezogen auf (2) eingesetzt und dient als Lösungs- bzw. Dispersionsmittel, die untere Grenze der Alkoholmenge wird im allgemeinen von der Rührbarkeit der Reaktionslösung bestimmt, doch kann die Menge gegebenenfalls auf etwa 2 mol pro mol 4-Amino-2--carboxymethylthioverbindung (2) reduziert werden. Die Konzentration der Reaktionslösung sollte möglichst gross sein.

Bevorzugte Alkohole sind Methanol, Äthanol, Propanol und Butanol.

Als Base wird bevorzugt das dem Alkohol entsprechende Alkoholat vorteilhaft Natrium- oder Kaliumalkoholat verwendet, doch können auch andere Alkoholate wie z.B. Erdalkalialkoholate eingesetzt werden. Als weitere Basen sind besonders die Alkalihydroxide oder gegebenenfalls Erdalkalihydroxide, -oxide oder Salze schwacher Säuren, wie z.B. Carbonate, geeignet. Die Alkalimenge beträgt 2 bis 20, vorzugsweise 2 bis 10 mol pro mol 4-Amino-2-carboxymethylthiopyrimidin bzw. 1 bis 20, vorzugsweise 2 bis 10 mol pro mol 4-Amino-2-alkoxycarbonylmethylthiopyrimidin.

Die Reaktion erfordert erhöhte Temperaturen. Bevorzugte Temperatur ist die Siedetemperatur des Lösungsmittels, doch sind auch höhere Temperaturen bis etwa 150°C unter geringem Überdruck möglich. Die Reaktionszeit beträgt im allgemeinen 1 bis 10 Stunden.

Die Aufarbeitung des Reaktionsproduktes ist verschieden in Abhängigkeit von den Produkteigenschaften. So kann das schwer wasserlösliche 4-Amino-2-methoxypyrimidin nach Reaktionsende und Abdestillieren des Methanols durch Zugabe von Wasser ausgefällt und von den wasserlöslichen Bestandteilen abgetrennt werden.

4-Amino-2-alkoxypyrimidine sind Ausgangsprodukte z.B. für pharmazeutisch wirksame Sulfonamide und für Cytosin und Cytosinderivate.

*Beispiel 1*

37 g (0,2 mol) 4-Amino-2-carboxymethylthiopyrimidin und 108 g einer 30 proz. methanolischen Natriummethylatlösung (0,6 mol Natriummethylat) werden bei Raumtemperatur gemischt und 3 Stunden zum Rückfluss erhitzt. Nach dem Abziehen des Lösungsmittels im Vakuum wird der Rückstand mit 150 ml Wasser versetzt und ca. 1 Stunde bei 50 bis 60°C gerührt. Die Lösung wird auf 20°C abgekühlt, der Feststoff abgetrennt, mit Wasser gewaschen und im Vakuum getrocknet.

Ausbeute: 22,3 g (89,2% d.Th.) 4-Amino-2--methoxypyrimidin.

Schmp.: 166-168°C.

*Beispiel 2*

9,3 g (0,05 mol) 4-Amino-2-carboxymethylthiopyrimidin wird mit 6,6 g (0,165 mol) Natriumhydroxid in 40 ml Methanol 3 Stunden zum Rückfluss erhitzt. Nach der Aufarbeitung wie in Beispiel 1 werden 4,7 g (75,2% d.Th.) 4-Amino-2-methoxypyrimidin erhalten.

*Beispiel 3*

9,3 g (0,05 mol) 4-Amino-2-carboxymethylthiopyrimidin wird in 120 g einer 17 proz. äthanolischen Natriumäthylatlösung (0,3 mol Natriumäthylat) 3 Stunden zum Rückfluss erhitzt. Das Lösungsmittel wird im Vakuum abgezogen, der Rückstand in 50 ml Wasser aufgenommen, mit Äther extrahiert und nach dem Trocknen der Äther abgezogen. Der Rückstand wird aus Äther/Petroläther umkristallisiert.

Ausbeute: 4,8 g (69,1% d.TH.) 4-Amino-2-äthoxypyrimidin.

Schmp.: 72-76°C.

*Beispiel 4*

19,9 g (0,1 mol) 4-Amino-2-methoxycarbonylmethylthiopyrimidin wird in 81 g einer 30 proz. methanolischen Natriummethylatlösung (0,45 mol Natriummethylat) 3 Stunden zum Rückfluss erhitzt. Nach Aufarbeitung analog Beispiel 1 werden 9,5 g (76,0% d.Th.) 4-Amino-2-methoxypyrimidin erhalten.

*Beispiel 5*

1,0 g (0,005 mol) 4-Amino-5-methyl-2-carboxymethylthiopyrimidin wird in 7,2 g einer 17 proz. methanolischen Natriummethylatlösung (0,0225 mol) 3 Stunden zum Rückfluss erhitzt. Nach Aufarbeitung analog Beispiel 3 werden 0,31 g (44,6% d.Th.) 4-Amino-2-methoxy-5-methylpyrimidin erhalten.

Schmp.: 103-107°C.

*Beispiel 6*

5,5 g (0,02 mol) 4-Amino-5-benzyl-2-carboxymethylthiopyrimidin wird in 28,6 g methanolischer Natriummethylatlösung 3 Stunden zum Rückfluss erhitzt. Nach Aufarbeitung analog Beispiel 1 werden 3,1 g (72,1% d.Th.) 4-Amino-5-benzyl-2-methoxypyrimidin erhalten.

Schmp.: 140-141°C.

*Beispiel 7*

Entsprechend Beispiel 1 werden 42,8 g (0,2 mol) 4-Amino-5-methoxy-methyl-2-carboxymethylfluopyrimidin umgesetzt und in entsprechender Ausbeute 4-Amino-5-methoxymethyl-2-methoxypyrimidin erhalten.

**Patentansprüche**

1. Verfahren zur Herstellung von 2-Alkoxy-4-aminopyrimidinen der Formel

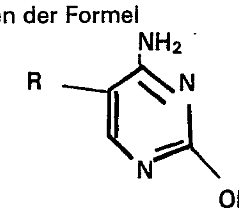

1,

worin R ein Wasserstoffatom, geradkettige, verzweigte oder cyclische Alkylreste mit 1 bis 20 Kohlenstoffatomen, geradkettige oder verzweigte Reste $(CH_2)_nCN$, $(CH_2)_n$-COOR″, $(CH_2)_n$-$NH_2$, $(CH_2)_nOR''$, $(CH_2)_n$-Cyc, wobei Cyc ein isocyclischer oder heterocyclischer Ring mit ein- oder mehrcyclischer Struktur, R″ Alkylreste mit 1 bis 12 Kohlenstoffatomen oder Reste einwertiger Phenole und n = 1 bis 5 ist, und R′ geradkettige, verzweigte oder cyclische Alkylreste mit 1 bis 10 Kohlenstoffatomen oder $(CH_2)_n$Cyc, wobei n und Cyc die oben angegebene Bedeutung haben, bedeutet, dadurch gekennzeichnet, dass ein 4-Amino-2-carboxymethylthiopyrimidin der Formel

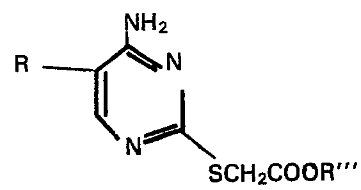

2,

worin R dieselbe Bedeutung wie in der Formel (1) hat und R‴ Wasserstoff oder einen geradkettigen, verzweigten oder cyclischen Alkylrest mit 1 bis 10 Kohlenstoffatomen bedeutet, mit einem Alkohol der Formel

HOR′ 3,

worin R′ dieselbe Bedeutung wie in der Formel (1) besitzt, in Gegenwart einer Base umgesetzt wird.

2. Verfahren nach Anspruch 1, dadurch gekennzeichnet, dass der Alkohol im Überschuss eingesetzt wird.

3. Verfahren nach einem der Ansprüche 1 oder 2, dadurch gekennzeichnet, dass als Basen Alkali- oder Erdalkalialkoholate, oder Alkali- oder Erdalkalihydroxide eingesetzt werden.

**Claims**

1. Process for the preparation of 2-alkoxy-4-aminopyrimidines of the formula

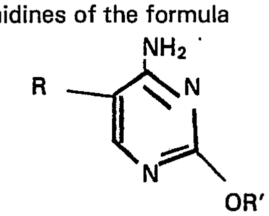

1,

wherein R denotes a hydrogen atom, straight-chained, branched or cyclic alkyl residues with 1 to 20 carbon atoms, straight-chained or branched re-

sidues $(CH_2)_nCN$, $(CH_2)_n\text{-}COOR''$, $(CH_2)_n\text{-}NH_2$, $(CH_2)_nOR''$, $(CH_2)_n\text{-}Cyc$, wherein Cyc is an isocyclic or heterocyclic ring with mono- or polycyclic structure, R'' alkyl residues with 1 to 12 carbon atoms or residues of monovalent phenols and n = 1 to 5, and R' denotes straight-chained, branched or cyclic alkyl residues with 1 to 10 carbon atoms or $(CH_2)_nCyc$, wherein n and Cyc have the above-indicated meaning, characterised in that a 4-amino-2-carboxymethylpyrimidine of the formula

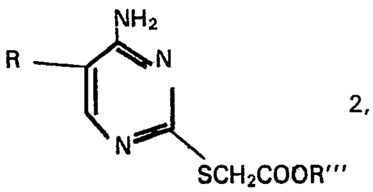

2,

wherein R has the same meaning as in formula (1) and R''' denotes hydrogen or a straight-chained, branched or cyclic alkyl residue with 1 to 10 carbon atoms, is reacted in the presence of a base with an alcohol of the formula

HOR' 3,

wherein R' possesses the same meaning as in formula (1).

2. Process according to claim 1, characterised in that the alcohol is employed in excess.

3. Process according to one of claims 1 or 2, characterised in that alkali or alkaline earth alcoholates, or alkali or alkaline earth hydroxides are employed as bases.

**Revendications**

1. Procédé pour la préparation d'alcoxy-2-amino-4 pyrimidine de formule

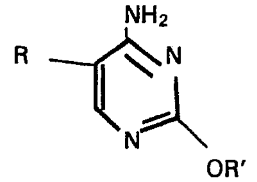

1,

dans laquelle R représente un atome d'hydrogène, des radicaux alkyles linéaires, ramifiés ou cycliques ayant 1 à 20 atomes de carbone, des radicaux linéaires ou ramifiés $(CH_2)_nCN$, $(CH_2)_n\text{-}COOR''$, $(CH_2)_n\text{-}NH_2$, $(CH_2)_nOR''$, $(CH_2)_n\text{-}Cyc$, Cyc étant un radical isocyclique ou hétérocyclique avec une structure à un ou plusieurs cycles, R'' des radicaux alkyles ayant 1 à 12 atomes de carbone ou des radicaux de monophénols et n = 1 à 5, et R' représente des radicaux alkyles linéaires, ramifiés ou cycliques ayant 1 à 10 atomes de carbone ou$(CH_2)_nCyc$, n et Cyc ayant la signification indiquée ci-dessus, caractérisé en ce que l'on fait réagir une amino-4 carboxyméthylthio-2 pyrimidine de formule

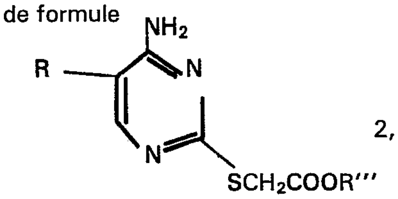

2,

dans laquelle R a la même signification que dans la formule (1) et R'' représente l'hydrogène ou un radical alkyle linéaire, ramifié ou cyclique ayant 1 à 10 atomes de carbone, avec un alcool de formule

HOR' 3,

dans laquelle R' a la même signification que dans la formule (1) en présence d'une base.

2. Procédé selon la revendication 1, caractérisé en ce que l'alcool est employé en excès.

3. Procédé selon la revendication 1 ou 2, caractérisé en ce que l'on emploie comme base des alcoolates de métaux alcalins ou alcalino-terreux ou des hydroxydes de métaux alcalins ou alcalino-terreux.